# EUROPÄISCHE PATENTANMELDUNG

(11) **EP 4 603 859 A1**
(43) Veröffentlichungstag der Anmeldung: **20.08.2025**
(21) Anmeldenummer: 24157310.4
(22) Anmeldetag: 13.02.2024
(51) Int. Cl.: G01R 33/563, A61B 5/00

(54) **MAGNETRESONANZSYSTEM UMFASSEND EINE MAGNETRESONANZVORRICHTUNG UND EINE ELASTOGRAPHIEVORRICHTUNG**

(71) Anmelder: Siemens Healthineers AG, 91301 Forchheim (DE)
(72) Erfinder: Sukkau, Johann, 91074 Herzogenaurach (DE)
(74) Vertreter: Siemens Healthineers Patent Attorneys

(57) **Zusammenfassung**

Die Erfindung geht aus von einer Magnetresonanzsystem umfassend eine Magnetresonanzvorrichtung mit einer Scannereinheit, die einen Grundmagneten, eine Gradientenspuleneinheit und eine Hochfrequenzspuleneinheit aufweist, und einem von der Scannereinheit zumindest teilweise umgebenen Patientenaufnahmebereich, und eine Elastographievorrichtung, die zu einer Anregung von zu untersuchenden Bereichen eines Patienten während einer Magnetresonanz-Elastographie-Untersuchung am Patienten ausgebildet ist, umfassend eine Vibrationseinheit, eine magnetresonanzkompatiblen Antriebseinheit und eine Übertragungseinheit zu einem Übertragen eines von der magnetresonanzkompatiblen Antriebseinheit erzeugten Antriebsmoments auf die Vibrationseinheit, wobei die magnetresonanzkompatible Antriebseinheit einen magnetresonanzkompatiblen Motor aufweist.

## Beschreibung

Die vorliegende Erfindung betrifft ein Magnetresonanzsystem umfassend eine Magnetresonanzvorrichtung mit einer Scannereinheit, die einen Grundmagneten, eine Gradientenspuleneinheit und eine Hochfrequenzantenneneinheit aufweist, und einem von der Scannereinheit zumindest teilweise umgebenen Patientenaufnahmebereich. Des Weiteren umfasst das Magnetresonanzsystem eine Elastographievorrichtung, die zu einer Anregung von zu untersuchenden Bereichen eines Patienten während einer Magnetresonanz-Elastographie-Untersuchung am Patienten ausgebildet ist, umfassend eine Vibrationseinheit, eine magnetresonanzkompatible Antriebseinheit und eine Übertragungseinheit zu einem Übertragen eines von der magnetresonanzkompatiblen Antriebseinheit erzeugten Antriebsmoments auf die Vibrationseinheit.

Unabhängig vom grammatikalischen Geschlecht eines bestimmten Begriffes sind Personen mit männlicher, weiblicher oder anderer Geschlechteridentität mit umfasst.

Gesundes Gewebe und Tumorgewebe oder krankes Gewebe, beispielsweise eine an Leberfibrose erkrankte Leber, weisen unterschiedliche Vibrationseigenschaften und/oder ein unterschiedliches Anregungsverhalten bei einer Vibrationsanregung auf. Die diagnostische Bildgebung nutzt bei der Elastographie die unterschiedlichen Vibrationseigenschaften und/oder das unterschiedliche Anregungsverhalten zwischen den unterschiedlichen Gewebearten, insbesondere einem gesunden Gewebe und einem kranken Gewebe oder Tumorgewebe. Dieses unterschiedliche Verhalten kann mittels der Magnetresonanzbildgebung in einem Magnetresonanz-Elastogramm dargestellt werden.

In Verbindung mit der Magnetresonanztomographie kann dabei beispielsweise ein Schwingungsverhalten des Lebergewebes des

Patienten mittels einer Magnetresonanzmessung erfasst werden und daraus ein Zustand der Leber abgeleitet werden. Jedoch sind mit der Magnetresonanztomographie besondere Anforderungen an die Elastographievorrichtung verbunden. Beispielsweise muss eine Antriebseinheit, beispielsweise ein Motor, zur Erzeugung eines Antriebsmoments für die Elastographievorrichtung außerhalb einer Scannereinheit, insbesondere eines Patientenaufnahmebereichs der Scannereinheit, angeordnet sein. Dies wiederum erfordert eine lange Übertragungswelle zur Übertragung des Antriebsmoments auf eine Vibrationseinheit der Elastographievorrichtung.

Der vorliegenden Erfindung liegt insbesondere die Aufgabe zugrunde, eine besonders kompakte und platzsparende Elastographievorrichtung für Magnetresonanz-Elastographie-Untersuchungen bereitzustellen. Die Aufgabe wird durch die Merkmale des unabhängigen Anspruchs gelöst. Vorteilhafte Ausgestaltungen sind in den Unteransprüchen beschrieben.

Die Erfindung geht aus von einem Magnetresonanzsystem umfassend:
- eine Magnetresonanzvorrichtung mit einer Scannereinheit, die einen Grundmagneten, eine Gradientenspuleneinheit und eine Hochfrequenzantenneneinheit aufweist, und einem von der Scannereinheit zumindest teilweise umgebenen Patientenaufnahmebereich, und
- eine Elastographievorrichtung, die zu einer Anregung von zu untersuchenden Bereichen eines Patienten während einer Magnetresonanz-Elastographie-Untersuchung ausgebildet ist, umfassend eine Vibrationseinheit, eine magnetresonanzkompatible Antriebseinheit und eine Übertragungseinheit zu einem Übertragen eines von der magnetresonanzkompatiblen Antriebseinheit erzeugten Antriebsmoments auf die Vibrationseinheit.

Erfindungsgemäß weist die magnetresonanzkompatible Antriebseinheit einen magnetresonanzkompatiblen Motor auf.

Das Magnetresonanzsystem mit der Magnetresonanzvorrichtung und der Elastographievorrichtung ist bevorzugt dazu ausgelegt, unterschiedliche Vibrationseigenschaften und/oder ein unterschiedliches Anregungsverhalten von unterschiedlichen Gewebearten, wie beispielsweise von gesundem Gewebe und Tumorgewebe oder krankem Gewebe in einem Magnetresonanz-Elastogramm darzustellen. Hierzu wird mittels der Elastographievorrichtung ein zu untersuchender Bereich des Patienten mittels Vibrationen und/oder Schwingungen angeregt. Das unterschiedliche Schwingungsverhalten und/oder Vibrationsverhalten der unterschiedlichen Gewebearten wird mittels der Magnetresonanzbildgebung in einem Magnetresonanz-Elastogramm dargestellt.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu die Scannereinheit. Die Scannereinheit umfasst bevorzugt eine Magneteinheit zur Erfassung der medizinischen und/oder diagnostischen Bilddaten. Vorteilhafterweise umfasst hierbei die Scannereinheit, insbesondere die Magneteinheit, einen Grundmagneten, ein Gradientenspuleneinheit und eine Hochfrequenzantenneneinheit. Die Hochfrequenzantenneneinheit ist fest innerhalb der Scannereinheit angeordnet und zur Aussendung eines Anregungspulses ausgelegt und/oder ausgebildet.

Der Grundmagnet ist zur Erzeugung eines homogenen Grundmagnetfelds mit einer definierten Magnetfeldstärke, wie beispielsweise mit einer Magnetfeldstärke von 3 T oder 1,5 T usw., ausgebildet. Insbesondere ist der Grundmagnet zur Erzeugung eines starken und konstanten Grundmagnetfelds ausgebildet. Das homogene Grundmagnetfeld ist bevorzugt innerhalb des Patientenaufnahmebereichs der Magnetresonanzvorrichtung angeordnet und/oder vorzufinden. Die Gradientenspuleneinheit ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt und/oder ausgebildet. Der Patientenaufnahmebereich umfasst bevorzugt den Bereich, der dem Patienten während einer Magnetresonanzuntersuchung zur Verfügung steht. Beispielsweise ist hierzu der Patientenaufnahmebereich zylinderförmig ausgebildet und/oder zylinderförmig von der Scannereinheit, insbesondere der Magneteinheit, der Magnetresonanzvorrichtung umgeben. Insbesondere umfasst hierbei die Scannereinheit, insbesondere die Magneteinheit, eine den Patientenaufnahmebereich zumindest teilweise umgebende Umhausung. Bevorzugt umgibt dabei die Umhausung den Patientenaufnahmebereich zylinderförmig. Die Umhausung kann dabei einstückig mit der Hochfrequenzantenneneinheit der Magneteinheit ausgebildet sein und eine dem Patientenaufnahmebereich zugewandte Seite der Hochfrequenzantenneneinheit umfassen.

Innerhalb des Patientenaufnahmebereichs ist bevorzugt ein Field of View (FoV) und/oder ein Isozentrum der Magnetresonanzvorrichtung angeordnet. Das FoV umfasst bevorzugt einen Erfassungsbereich der Magnetresonanzvorrichtung, innerhalb dessen die Bedingungen für eine Erfassung von medizinischen Bilddaten, insbesondere von Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten aufweist. Insbesondere umfasst das Isozentrum den homogensten Magnetfeldbereich innerhalb der Magnetresonanzvorrichtung.

Die Vibrationseinheit der Elastographievorrichtung ist dazu ausgebildet, Vibrationen und/oder Druckwellen zu erzeugen und diese auf den Patienten, insbesondere auf einen zu untersuchenden Bereich des Patienten, zu übertragen. Hierzu ist bevorzugt die Vibrationseinheit in einer Nähe zum Patienten, insbesondere in einer Nähe des zu untersuchenden Bereichs des Patienten, positioniert. Insbesondere wird hierbei die Vibrationseinheit an den zu untersuchenden Bereich am Patienten angelegt. Zur Positionssicherung der Vibrationseinheit kann diese auch mit Gurten am Patienten befestigt und/oder fixiert werden. Die Vibrationseinheit befindet sich somit innerhalb des Patientenaufnahmebereichs während der Untersuchung.

Unter einer magnetresonanzkompatiblen Antriebseinheit, insbesondere einem magnetresonanzkompatiblen Motor, soll bevorzugt eine Antriebseinheit, insbesondere ein Motor, zur Verwendung mit einer Magnetresonanzvorrichtung verstanden werden, wobei die Antriebseinheit, insbesondere der Motor, nicht bildgebend ausgebildet ist. Insbesondere weist hierbei die magnetresonanzkompatible Antriebseinheit, insbesondere der magnetresonanzkompatible Motor, keine bildgebend ausgebildeten Komponenten auf, so dass eine Beeinträchtigung einer Magnetresonanzmessung vorteilhaft verhindert werden kann.

Die Übertragungseinheit der Elastographievorrichtung umfasst bevorzugt eine Antriebswelle zur Übertragung des Antriebsmoments, insbesondere eines Drehmoments, von der magnetresonanzkompatiblen Antriebseinheit, insbesondere dem magnetresonanzkompatiblen Motor, auf die Vibrationseinheit. Bevorzugt ist auch die Übertragungseinheit, insbesondere die Antriebswelle, magnetresonanzkompatibel, insbesondere nicht bildgebend, ausgebildet. Die Übertragungseinheit, insbesondere die Antriebswelle, ist bevorzugt biegsam ausgebildet, um beispielsweise auch unterschiedliche Anatomien von unterschiedlichen Patienten bei der Anordnung und/oder Positionierung der Elastographievorrichtung zu berücksichtigen. Dabei kann die Antriebswelle eine biegsame Welle umfassen. Alternativ oder zusätzlich kann die Übertragungseinheit, insbesondere die Antriebswelle, auch zumindest ein Gelenk, insbesondere ein Kardangelenk, aufweisen oder auch als Kardanwelle ausgebildet sein. Vorzugsweise ist die Übertragungseinheit, insbesondere die Antriebswelle, auch umhüllt, beispielsweise mit einem Schlauch, so dass eine Verletzung des Patienten während einer Übertragung eines Antriebsmoments, insbesondere eines Drehmoments, auf die Vibrationseinheit vorteilhaft verhindert wird.

Die Elastographievorrichtung kann zudem auch eine Kopplungseinheit umfassen, mittels der die Elastographievorrichtung mit der Magnetresonanzvorrichtung hinsichtlich eines Datenaustauschs gekoppelt und/oder verbunden ist. Beispielsweise können mittels der Kopplungseinheit Steuersignale zwischen der Magnetresonanzvorrichtung und der Elastographievorrichtung ausgetauscht werden. Die Kopplungseinheit kann dabei eine kabelgebundene Kopplungseinheit umfassen. Zudem ist auch eine kabellose und/oder eine drahtlose Kopplungseinheit jederzeit möglich. Bevorzugt umfasst die Kopplungseinheit eine Datenübertragungseinheit zwischen einer Steuereinheit der Elastographievorrichtung und einer Steuereinheit der Magnetresonanzvorrichtung.

Durch die Erfindung kann vorteilhaft eine besonders platzsparende und kompakte Elastographievorrichtung für Magnetresonanz-Elastographie-Untersuchungen bereitgestellt werden. Besonders vorteilhaft kann die magnetresonanzkompatible Antriebseinheit, insbesondere der magnetresonanzkompatible Motor, besonders nah an den zu untersuchenden Bereich des Patienten und damit an der Vibrationseinheit positioniert werden, so dass eine kurze Übertragungseinheit, insbesondere eine kurze Antriebswelle, zur Übertragung des Antriebsmoments verwendet werden kann.

In einer vorteilhaften Weiterbildung des Magnetresonanzsystems kann es vorgesehen sein, dass der magnetresonanzkompatible Motor innerhalb eines von dem Grundmagneten erzeugten homogenen Grundmagnetfelds angeordnet ist, wobei der magnetresonanzkompatible Motor einen Stator umfasst und der Stator eine dominante Komponente des Grundmagnetfelds umfasst. Der magnetresonanzkompatible Motor umfasst dabei einen elektromagnetischen Motor, dessen Stator die dominante Komponente des Grundmagnetfelds der Magnetresonanzvorrichtung umfasst. Innerhalb des Patientenaufnahmebereichs und/oder nahe am Isozentrum umfasst das Grundmagnetfeld des Grundmagneten nur eine dominante Komponente B₀ in z-Richtung der Magnetresonanzvorrichtung. Die z-Richtung der Magnetresonanzvorrichtung ist dabei parallel zu einer Längsrichtung des Patientenaufnahmebereichs ausgerichtet. Auch außerhalb des FoVs und/oder außerhalb des Patientenaufnahmebereichs ist die dominante Komponente des Grundmagnetfelds und/oder eines Streufelds bevorzugt in z-Richtung der Magnetresonanzvorrichtung ausgerichtet. Diese dominante Komponente des Grundmagnetfelds dient der magnetresonanzkompatiblen Antriebseinheit, insbesondere dem elektromagnetischen magnetresonanzkompatiblen Motor, als Stator. Bevorzugt ist hierbei die dominanten Komponenten des Grundmagnetfelds senkrecht zu einer Motorachse der magnetresonanzkompatiblen Antriebseinheit ausgerichtet.

Bevorzugt umfasst der magnetresonanzkompatible Motor einen Rotor und/oder ein drehbares Motorelement. Der Rotor und/oder das drehbare Motorelement umfasst zumindest ein drehbar gelagertes Spulenelement mit einer senkrecht zur dominanten Komponente des Grundmagnetfelds ausgerichteten Spulenachse. Das drehbar gelagerte Motorelement ist zur Generierung eines Antriebsmoments des magnetresonanzkompatiblen Motors ausgebildet. Eine Drehbewegung des drehbar gelagerten Motorelements kann dabei auch nur eine Teildrehung und keine komplette Drehung um die Motorachse umfassen. Vorzugsweise ist das zumindest eine drehbar gelagerte Motorelement in beide Richtungen um die Spulenachse drehbar gelagert. Das drehbar gelagerte Motorelement weist zumindest eine Spulenwicklung und bevorzugt mehrere Spulenwicklungen auf, so dass eine große Kraft, insbesondere eine große Lorentzkraft, auf das zumindest eine drehbar gelagerte Motorelement zur Generierung eines Antriebsmoments wirken kann. Besonders vorteilhaft weist das zumindest eine drehbar gelagerte Motorelement, insbesondere das drehbar gelagerte Spulenelement, des magnetresonanzkompatiblen Motors eine Kupferdrahtspule mit mehreren Spulenwicklungen auf. Bei einer Drehung des zumindest einen drehbar gelagerten Motorelements um die Spulenachse ändert sich dabei eine Neigung der Spulenfläche bezüglich des Grundmagnetfelds und/oder der dominanten Komponente des Grundmagnetfelds des Grundmagneten. Eine Drehrichtung und/oder Rotationsrichtung des drehbar gelagerten Spulenelements ist dabei abhängig von einer Stromrichtung eines durch das drehbar gelagerten Spulenelement fließenden Stroms. So kann durch Änderung der Stromrichtung auch eine Drehrichtung und/oder Rotationsrichtung geändert werden.

Eine weitere Ausgestaltung des magnetresonanzkompatiblen Motors, genauer eines Schrittmotors, entspricht den Ausführungen zu dem magnetresonanzkompatiblen Schrittmotor in der Patentschrift DE 10 2020 211 326 A1, auf die hiermit explizit verwiesen wird.

Diese Ausgestaltung der Erfindung weist den Vorteil auf, dass ein Antriebsmoment des magnetresonanzkompatiblen Motors und damit für eine Erzeugung von Vibrationen und/oder Druckwellen zur Anregung eines zu untersuchenden Bereichs und/oder Gewebes des Patienten besonders einfach generiert werden kann.

In einer vorteilhaften Weiterbildung des Magnetresonanzsystems kann es vorgesehen sein, dass der Grundmagnet zumindest eine Magnetspule zur Erzeugung des homogenen Grundmagnetfelds aufweist, wobei der magnetresonanzkompatible Motor zur Erzeugung des Antriebsmoments für die Vibrationseinheit innerhalb des Patientenaufnahmebereichs in einem von der Magnetspule überdeckten Bereich angeordnet ist. Bevorzugt umfasst der Grundmagnet ein Helmholtz-Spulenpaar mit zwei Magnetspulen. Dabei ist eine erste Magnetspule des Helmholtz-Spulenpaares in einem Frontbereich der Magneteinheit, insbesondere des Grundmagneten, und eine zweite Magnetspulen des Helmholtz-Spulenpaares in einem Heckbereich der Magneteinheit, insbesondere des Grundmagneten, angeordnet. Der Frontbereich erstreckt sich hierbei 10 cm bis 30 cm von einer Einführöffnung des Patientenaufnahmebereich in den Patientenaufnahmebereich und die Magneteinheit hinein. Der Heckbereich erstreckt sich hierbei 10 cm bis 30 cm von einer Endöffnung des Patientenaufnahmebereich in den Patientenaufnahmebereich und die Magneteinheit hinein.

Zwischen den beiden Magnetspulen des Helmholtz-Spulenpaares ist bevorzugt das FoV der Magneteinheit mit dem homogenen Grundmagnetfeld angeordnet. Dagegen ist an den Positionen, insbesondere in z-Richtung, der beiden Magnetspulen des Helmholtz-Spulenpaares das Magnetfeld maximal ausgebildet. Der magnetresonanzkompatible Motor ist bevorzugt in z-Richtung an einer gleichen Position angeordnet wie eine Magnetspule des Helmholtz-Spulenpaares, so dass der magnetresonanzkompatible Motor in einem von der Magnetspule überdeckten Bereich angeordnet ist. Für eine einfache Positionierung des magnetresonanzkompatiblen Motors innerhalb des Patientenaufnahmebereichs und damit innerhalb des Grundmagnetfelds während einer Magnetresonanz-Elastographie-Untersuchung ist der magnetresonanzkompatible Motor an einer Position der ersten Magnetspule des Helmholtz-Spulenpaares in der Nähe zur Eingangsöffnung angeordnet, so dass eine einfache Erreichbarkeit für einen Benutzer erreicht werden kann. Diese Ausgestaltung der Erfindung weist den Vorteil auf, dass der magnetresonanzkompatible Motor in einem maximalen Magnetfeld angeordnet werden kann und damit einen der magnetresonanzkompatible Motor mit einer geringen Stromstärke zur Erzeugung eines definierten Antriebsmoments betrieben werden kann.

In einer vorteilhaften Weiterbildung des Magnetresonanzsystems kann es vorgesehen sein, dass der magnetresonanzkompatible Motor einen Magnetfeldsensor aufweist. Der Magnetfeldsensor ist bevorzugt dazu ausgebildet, eine Größe des Grundmagnetfelds, insbesondere eine Größe der dominanten Komponente des Grundmagnetfelds, an der Position des magnetresonanzkompatiblen Motors zu bestimmen und/oder zu erfassen. Bevorzugt umfasst hierbei der Magnetfeldsensor einen Hallsensor zur Erfassung einer Größe des Grundmagnetfelds. Diese Ausgestaltung der Erfindung weist den Vorteil auf, dass eine exakte Größe des Grundmagnetfelds an der Position des Motors während einer Magnetresonanz-Elastographie-Untersuchung erfasst wird. Ausgehend von der exakten Größe des Grundmagnetfelds kann durch Einstellen einer entsprechenden Stromstärke für den magnetresonanzkompatiblen Motor ein definiertes Antriebsmoment, insbesondere Drehmoment, für die Vibrationseinheit generiert und/oder erzeugt werden. Bevorzugt erfolgt eine Einstellung einer Stromstärke an dem magnetresonanzkompatiblen Motor mittels einer Motortreibereinheit und einer Steuereinheit der Elastographievorrichtung.

In einer vorteilhaften Weiterbildung des Magnetresonanzsystems kann es vorgesehen sein, dass die Elastographievorrichtung eine Haltevorrichtung aufweist, an der der magnetresonanzkompatible Motor während einer Magnetresonanz-Elastographie-Untersuchung angeordnet ist. Bevorzugt weist die Haltevorrichtung zumindest ein Befestigungselement auf, wobei mittels des Befestigungselements der magnetresonanzkompatible Motor an der Haltevorrichtung befestigt werden kann. Vorteilhafterweise ist die Haltevorrichtung derart ausgebildet, dass der magnetresonanzkompatible Motor in einer definierten Position bezüglich der dominanten Komponente des Grundmagnetfelds, insbesondere mit einer Spulenachse senkrecht zur dominanten Komponente des Grundmagnetfelds, an der Haltevorrichtung während einer Magnetresonanz-Elastographie-Untersuchung angeordnet ist. Derart kann eine sichere und stabile Positionierung des magnetresonanzkompatiblen Motors während einer Magnetresonanz-Elastographie-Untersuchung vorteilhaft erreicht werden. Insbesondere behält der magnetresonanzkompatible Motor seine Position während einer Magnetresonanz-Elastographie-Untersuchung bezüglich der dominanten Komponente des Grundmagnetfelds bei, so dass die einmal gewählten Einstellungen zur Generierung und/oder Erzeugung eines definierten Antriebsmoments für die Vibrationseinheit unverändert erhalten bleiben können. Zudem kann ein Verrutschen und/oder eine Fehlpositionierung des magnetresonanzkompatiblen Motors , wie dies beispielsweise bei einer Auflage des magnetresonanzkompatiblen Motors auf dem Patienten während einer Magnetresonanz-Elastographie-Untersuchung der Fall sein kann, vorteilhaft verhindert werden.

In einer vorteilhaften Weiterbildung des Magnetresonanzsystems kann es vorgesehen sein, dass die Magnetresonanzvorrichtung eine Patientenlagerungsvorrichtung mit einem innerhalb des Patientenaufnahmebereichs bewegbaren Patiententisch aufweist, wobei die Haltevorrichtung zu einer lösbaren Befestigung an dem Patiententisch ausgebildet ist.

Für eine Positionierung des Patienten, insbesondere des zu untersuchenden Bereichs des Patienten, innerhalb des Patientenaufnahmebereich weist die Magnetresonanzvorrichtung die Patientenlagerungsvorrichtung auf. Die Patientenlagerungsvorrichtung ist zu einer Lagerung des Patienten ausgebildet. Die Patientenlagerungsvorrichtung weist bevorzugt einen bewegbaren Patiententisch auf, der insbesondere innerhalb des Patientenaufnahmebereichs der Magnetresonanzvorrichtung bewegbar ausgebildet ist. Bevorzugt ist hierbei der Patiententisch in Längsrichtung des Patientenaufnahmebereichs und/oder in z-Richtung innerhalb des Patientenaufnahmebereichs bewegbar ausgebildet. Für eine Magnetresonanz-Elastographie-Untersuchung wird der Patient zunächst auf dem Patiententisch der Patientenlagerungsvorrichtung positioniert und die Elastographievorrichtung am Patienten und/oder am Patiententisch angeordnet und/oder positioniert. Zudem können auch weitere Zusatzeinheiten am Patienten positioniert werden, wie beispielsweise eine Injektionseinheit und/oder eine EKG-Einheit und/oder Positionierungskissen usw. Anschließend fährt der Patiententisch zusammen mit dem Patienten in den Patientenaufnahmebereich, bis der zu untersuchende Bereich des Patienten innerhalb des Isozentrums der Magnetresonanzvorrichtung angeordnet ist. Bevorzugt ist die Haltevorrichtung zu einer lösbaren Befestigung an dem Patiententisch ausgebildet, so dass ein einfaches Entfernen der Haltevorrichtung und damit auch des magnetresonanzkompatiblen Motors ermöglicht werden kann.

Diese Ausgestaltung der Erfindung weist den Vorteil auf, dass eine einfache und sichere Anordnung der Elastographievorrichtung, insbesondere des magnetresonanzkompatiblen Motors für eine Magnetresonanz-Elastographie-Untersuchung erreicht werden. Insbesondere kann derart die Haltevorrichtung sicher an dem Patiententisch für eine Magnetresonanz-Elastographie-Untersuchung angeordnet und/oder befestigt werden.

In einer vorteilhaften Weiterbildung des Magnetresonanzsystems kann es vorgesehen sein, dass die Haltevorrichtung einen konvexen Haltebogen mit zwei Endbereichen und einem mittleren Befestigungsbereich umfasst, wobei die beiden Endbereiche zu einer lösbaren Befestigung am Patiententisch ausgebildet sind und der mittlere Befestigungsbereich für eine Anordnung des magnetresonanzkompatiblen Motors ausgebildet ist. Bevorzugt weist der Patiententisch zwei Befestigungsschienen auf, die sich jeweils in Längsrichtung des Patiententischs erstrecken. Die beiden Befestigungsschienen sind dabei jeweils an einem seitlichen Randbereich des Patiententischs angeordnet, wobei zwischen den beiden seitlichen Randbereichen und damit den beiden Befestigungsschienen des Patiententischs eine Lagerungsfläche und/oder ein Lagerungsbereich des Patiententischs zur Lagerung des Patienten angeordnet ist. Mittels der beiden Befestigungsschienen können Zubehöreinheiten, die für eine anstehende Magnetresonanzuntersuchung und/oder eine anstehende Magnetresonanz-Elastographie-Untersuchung erforderlich sind, sicher am Patiententisch befestigt und/oder angeordnet werden.

Die beiden Endbereiche des konvexen Haltebogens sind zur Anordnung und/oder Befestigung an den beiden Befestigungsschienen des Patiententischs ausgebildet, wobei ein erster Endbereich in einer ersten der beiden Befestigungsschienen und zweiter Endbereich in einer zweiten der beiden Befestigungsschienen angeordnet ist. In einer befestigten Position am Patiententisch wölbt sich der konvexe Haltebogen über die Lagerungsfläche und/oder den Lagerungsbereich des Patiententischs. Dabei wölbt sich der konvexe Haltebogen von einer ersten Seite des Patiententischs bis zu einer zweiten Seite des Patiententischs, wobei zwischen den beiden Seiten die Lagerungsfläche des Patiententischs angeordnet ist. Somit wölbt sich der konvexe Haltebogen auch über den auf dem Patiententisch angeordneten Patienten, beispielsweise über einen Beinbereich des Patienten. Der konvexe Haltebogen ist in einer Draufsicht von oben auf den Patiententisch in einer an dem Patiententisch angeordneten Position konvex ausgebildet. In dem mittleren Bereich des konvexen Haltebogens ist der Befestigungsbereich zur Befestigung und/oder Anordnung des magnetresonanzkompatiblen Motors angeordnet. Bevorzugt weist der mittlere Befestigungsbereich des konvexen Haltebogens eine bevorzugte Befestigungsposition für den magnetresonanzkompatiblen Motor auf, so dass der magnetresonanzkompatible Motor in einer Position, bei der eine Motorachse des magnetresonanzkompatiblen Motors senkrecht zur dominanten Komponente des Grundmagnetfelds ausgerichtet ist, aufweist.

Diese Ausgestaltung der Erfindung ermöglicht eine sichere und stabile Anordnung des magnetresonanzkompatiblen Schrittmotors. Zudem ist durch diese Art der Anordnung des magnetresonanzkompatiblen Motors eine unerwünschte Beeinträchtigung des Patienten bei einer Positionierung auf dem Patiententisch vorteilhalft verhindert.

In einer vorteilhaften Weiterbildung des Magnetresonanzsystems kann es vorgesehen sein, dass die Magnetresonanzvorrichtung ein Positionierungskissen zu einer Positionierung eines Patienten aufweist, wobei der magnetresonanzkompatible Motor innerhalb des Positionierungskissens angeordnet ist. Das Positionierungskissen ist bevorzugt zur Positionierung und/oder bequemen Lagerung von Teilbereichen des Patienten ausgebildet, wie beispielsweise ein Positionierungskissen zur Positionierung und/oder Lagerung von Beinen oder Knieen des Patienten. Beispielsweise kann ein derartiges Positionierungskissen einen Aufnahmebereich und/oder eine Tasche aufweisen, die zur Aufnahme des magnetresonanzkompatiblen Motors ausgebildet ist. Bevorzugt weist der Aufnahmebereich und/oder die Tasche

des Positionierungskissen eine bevorzugte Befestigungsposition für den magnetresonanzkompatiblen Motor auf, so dass der magnetresonanzkompatible Motor in einer Position, bei der eine Motorachse des magnetresonanzkompatiblen Motors senkrecht zur dominanten Komponente des Grundmagnetfelds ausgerichtet ist, aufweist. Diese Ausgestaltung der Erfindung ermöglicht eine sichere und geschützte Anordnung des magnetresonanzkompatiblen Motors während einer Magnetresonanz-Elastographie-Untersuchung. Zudem ist durch diese Art der Anordnung des magnetresonanzkompatiblen Motors eine unerwünschte Beeinträchtigung des Patienten bei einer Positionierung auf dem Patiententisch vorteilhalft verhindert.

In einer vorteilhaften Weiterbildung des Magnetresonanzsystems kann es vorgesehen sein, dass die Übertragungseinheit ein in ihrer Länge variable Antriebswelle aufweist. Insbesondere kann die Länge der Antriebswelle von einem Benutzer bei einer Positionierung des Patienten und der Elastographievorrichtung auf dem Patiententisch eingestellt werden. Dies ermöglicht eine einfache Positionierung der Elastographievorrichtung am Patienten, da die magnetresonanzkompatible Antriebseinheit, insbesondere der magnetresonanzkompatible Motor, unabhängig von einer Länge der Übertragungseinheit, insbesondere der Antriebswelle, am Patienten und/oder am Patiententisch positioniert werden kann. Insbesondere kann derart eine Länge der Antriebswelle auch an eine Körpergröße des Patienten angepasst werden.

Um eine Beeinträchtigung der Magnetresonanzdatenerfassung während einer Magnetresonanz-Elastographie-Untersuchung zu vermeiden, sollte der magnetresonanzkompatible Motor einen Mindestabstand zu dem zu untersuchenden Bereich des Patienten aufweisen. Je nach Feldstärke des Grundmagneten der Magnetresonanzvorrichtung sollte dabei der Mindestabstand zwischen 4 cm und 90 cm betragen. Durch die in ihrer Länge variable Antriebswelle kann dieser Mindestabstand zwischen dem zu untersuchenden Bereich und dem magnetresonanzkompatiblen Motor eingehalten und zudem eine kompakte Elastographievorrichtung bereitgestellt werden.

Die in ihrer Länge variable Antriebswelle kann dabei teleskopartig ausgebildet sein. Beispielsweise kann die in ihrer Länge variable Antriebswelle zwei oder mehr ineinandergreifende Stangen, beispielsweise Stangen mit einem viereckigem oder einem sechseckigem Querschnitt, umfassen. Vorzugsweise weisen hierbei die ineinandergreifenden Stangen unterschiedliche Querschnitte auf.

In einer vorteilhaften Weiterbildung des Magnetresonanzsystems kann es vorgesehen sein, dass die Vibrationseinheit eine Vibrationselement aufweist, wobei das von dem magnetresonanzkompatiblen Motor erzeugte Antriebsmoment auf das Vibrationselement übertragbar ist. Das Vibrationselement umfasst bevorzugt eine Schwingmasse und/oder Vibrationsmasse, die zu einer Erzeugung einer Schwingung und/oder Vibration ausgebildet ist. Zudem ist das Vibrationselement dazu ausgebildet, die erzeugte Schwingung und/oder Vibration auf den Patienten, insbesondere auf den zu untersuchenden Bereich des Patienten zu übertragen. Durch Umpolung eines durch den magnetresonanzkompatiblen Motor fließenden Stroms kann ein Antriebsmoment, insbesondere ein Drehmoment, erzeugt werden, dass seine Richtung wechselt. Derart kann ein hin und her schwingendes Drehmoment generiert werden, das direkt auf das Vibrationselement, insbesondere auf die Schwingmasse, übertragen werden kann. Dies ermöglicht auch eine besonders kompakte Bauweise der Vibrationseinheit, da auf zusätzliche Vibrationserzeugerelemente vorteilhaft verzichtet werden kann.

Alternativ hierzu kann die Vibrationseinheit auch ein Exzenterelement aufweisen, wobei auf das Exzenterelement das von dem magnetresonanzkompatiblen Motor erzeugte Antriebsmoment übertragbar ist. Vorzugsweise umfasst die Vibrationseinheit zudem eine Sperrklinke, die zu dem Exzenterelement vorgeschaltet innerhalb der Vibrationseinheit angeordnet ist, so dass stets ein Drehmoment in eine gleiche Richtung auf das Exzenterelement für eine Generierung eine Vibration und/oder Schwingung übertragen wird.

In einer vorteilhaften Weiterbildung des Magnetresonanzsystems kann es vorgesehen sein, dass die Elastographievorrichtung eine Motortreibereinheit und ein Schirmgehäuse umfasst, wobei die Motortreibereinheit in dem Schirmgehäuse angeordnet ist. Die Motortreibereinheit umfasst insbesondere eine Schaltung und/oder eine Schaltungseinheit zur Ansteuerung des magnetresonanzkompatiblen Schrittmotors. Bevorzugt umfasst die Motortreibereinheit eine H-Brücke zur Spannungsregelung des magnetresonanzkompatiblen Schrittmotors. Das Schirmgehäuse schirmt die Motortreibereinheit von der Magneteinheit ab. Insbesondere schirmt das Schirmgehäuse die Motortreibereinheit hinsichtlich einer Hochfrequenzstrahlung von der Magneteinheit ab. Vorteilhaft umfasst das Schirmgehäuse ein elektromagnetisches Filterelement, wobei das elektromagnetische Filterelement alle ausgehenden Signale der Motortreibereinheit filtert, um Interaktionen der Magneteinheit zu vermeiden.

Aufgrund der Anordnung der Motortreibereinheit in dem Schirmgehäuse kann die Motortreibereinheit auch innerhalb des Patientenaufnahmebereichs und/oder in einem Bereich, in dem ein Streufeld des Grundmagnetfelds vorhanden ist, angeordnet werden. Derart kann eine unerwünschte Interaktion zwischen der Motortreibereinheit und der Scannereinheit vorteilhaft reduziert und/oder verhindert werden.

In einer vorteilhaften Weiterbildung des Magnetresonanzsystems kann es vorgesehen sein, dass die Elastographievorrichtung eine Steuereinheit aufweist, wobei die Steuereinheit zu einer Synchronisierung der Elastographievorrichtung mit einer Messsequenz der Magnetresonanz-Elastographie-Untersuchung ausgebildet ist.

Bevorzugt ist die Steuereinheit außerhalb des Patientenaufnahmebereichs der Scannereinheit angeordnet. Zudem weist die Steuereinheit eine Datenverbindung mit der Magnetresonanzvorrichtung, insbesondere einer Magnetresonanzsteuereinheit der Magnetresonanzvorrichtung, auf. Die Datenverbindung kann dabei kabelgebunden oder auch kabellos ausgebildet sein.

Die Steuereinheit der Elastographievorrichtung umfasst zumindest ein Rechenmodul und/oder einen Prozessor. So ist insbesondere die Steuereinheit dazu ausgebildet, computerlesbare Instruktionen auszuführen. Insbesondere umfasst die Steuereinheit eine Speichereinheit, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen. Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Steuereinheit sendet in einem Betriebsmodus der Elastographievorrichtung Steuersignale direkt an die Motortreibereinheit und steuert somit die Elastographievorrichtung. Bevorzugt steuert die Steuereinheit die Motortreibereinheit derart, dass ein Schwingungsprozess und/oder Anregungsprozess synchron mit der auszuspielenden Messsequenz der Magnetresonanz-Elastographie-Untersuchung ist. Hierdurch kann eine vorteilhafte Abstimmung zwischen der Magnetresonanzvorrichtung und der Elastographievorrichtung bereitgestellt werden. Bevorzugt ist hierbei ein Anfang und eine Ende der Messsequenz auf den Schwingungsprozess und/oder Anregungsprozess der Elastographievorrichtung abgestimmt. Zur Synchronisierung mit der Messsequenz kann die Steuereinheit der Elastographievorrichtung auch zumindest ein Triggersignal einer Magnetresonanzsequenzsteuereinheit bereitstellen, so dass die Messsequenz getriggert durch die Elastographievorrichtung gestartet wird. Dabei kann für die Elastographievorrichtung auch ein Einschwingvorgang vorgesehen sein, bis eine Mechanik, insbesondere der magnetresonanzkompatible Motor und die Vibrationseinheit, sich auf eine Anregungsfrequenz eingeschwungen haben. Dieser Einschwingvorgang wird bevorzugt von der Steuereinheit bei der Bereitstellung des Triggersignals berücksichtigt. Ein Anregungsvorgang und/oder ein Schwingungsvorgang für eine Anregung mittels der Elastographievorrichtung umfasst bevorzugt eine Frequenz zwischen 50 Hz und 1000 Hz. Bevorzugt umfasst eine Anregungsfrequenz und/oder eine Schwingungsfrequenz ca. 100 Hz.

Eine Messsequenz umfasst bevorzugt eine Magnetresonanzsequenz, wobei eine Magnetresonanzsequenz bevorzugt eine zeitliche Folge von Hochfrequenzpulsen umfasst. Beispielsweise kann eine Magnetresonanzsequenz eine T1-gewichtete Sequenz oder eine T2-gewichtete Sequenz oder eine Spin-Echo-Sequenz usw. umfassen. Die einzelnen Magnetresonanzsequenzen unterscheiden sich dabei hinsichtlich ihrer Sequenzparameter.

In einer vorteilhaften Weiterbildung des Magnetresonanzsystems kann es vorgesehen sein, dass die Elastographievorrichtung eine optische Übertragungseinheit umfasst, die zwischen der Motortreibereinheit und der Steuereinheit angeordnet ist. Die optische Verbindungseinheit umfasst bevorzugt optische Leiter, beispielsweise Lichtleiter und/oder Glasfaserkabel. Derart kann eine störungsfreie Signalübertragung zwischen der Motortreibereinheit und der Steuereinheit erreicht werden.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: ein erfindungsgemäßes Magnetresonanzsystem mit einer Magnetresonanzvorrichtung und einer Elastographievorrichtung in einer schematischen Darstellung,
- Fig. 2: eine Positionierung einer magnetresonanzkompatiblen Antriebseinheit der Elastographievorrichtung bezüglich eines Grundmagneten der Magnetresonanzvorrichtung,
- Fig. 3: ein Aufbau der Elastographievorrichtung,
- Fig. 4: ein Aufbau des magnetresonanzkompatiblen Motors in einer Schnittdarstellung,
- Fig. 5: ein erstes Ausführungsbeispiel der Elastographievorrichtung mit einer Haltevorrichtung,
- Fig. 6: ein zweites Ausführungsbeispiel der Elastographievorrichtung mit einer Anordnung der magnetresonanzkompatiblen Antriebseinheit in einem Positionskissen, und
- Fig. 7: das zweite Ausführungsbeispiel in einer Seitenansicht.

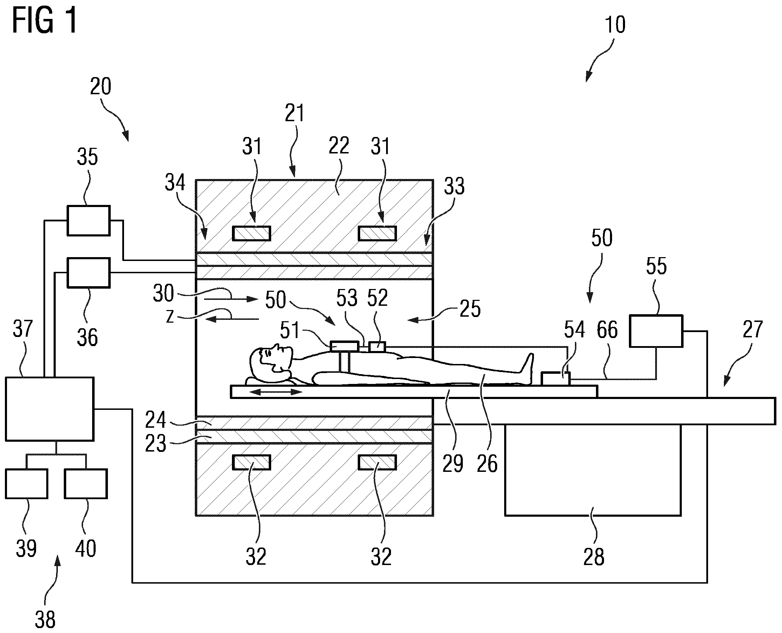

In der Fig. 1 ist ein Magnetresonanzsystem 10 mit einer Magnetresonanzvorrichtung 20 und einer Elastographievorrichtung 50 schematisch dargestellt. Die Magnetresonanzvorrichtung 20 umfasst eine als Magneteinheit 21 ausgebildete Scannereinheit. Die Scannereinheit, insbesondere die Magneteinheit 21, umfasst einen Grundmagneten 22, eine Gradientenspuleneinheit 23 und eine Hochfrequenzantenneneinheit 24. Zudem weist die Magnetresonanzvorrichtung 20 einen Patientenaufnahmebereich 25 auf zu einer Aufnahme eines Patienten 26 für eine Magnetresonanzuntersuchung und/oder eine Magnetresonanz-Elastographie-Untersuchung. Der Patientenaufnahmebereich 25 im vorliegenden Ausführungsbeispiel ist zylinderförmig ausgebildet und in einer Umfangsrichtung von der Scannereinheit, insbesondere der Magneteinheit 21, zylinderförmig umgeben. Grundsätzlich ist jedoch eine davon abweichende Ausbildung des Patientenaufnahmebereichs 25 jederzeit denkbar.

Für eine Positionierung des Patienten 26, insbesondere eines zu untersuchenden Bereichs des Patienten 26, innerhalb des Patientenaufnahmebereichs 25 weist die Magnetresonanzvorrichtung 20 eine Patientenlagerungsvorrichtung 27 auf. Die Patientenlagerungsvorrichtung 27 weist eine Basiseinheit 28 und einen bezüglich der Basiseinheit 28 bewegbaren Patiententisch 29 auf. Der Patiententisch 29 ist für eine Positionierung des Patienten 26, insbesondere des zu untersuchenden Bereichs des Patienten 26, bewegbar innerhalb des Patientenaufnahmebereichs 25 ausgebildet. Insbesondere ist hierbei der Patiententisch 29 in Richtung einer Längserstreckung des Patientenaufnahmebereichs 25 und/oder in z-Richtung bewegbar gelagert.

Der Grundmagnet 22 der Magneteinheit 21 ist zu einem Erzeugen eines starken und insbesondere konstanten Grundmagnetfelds 30 ausgebildet. Der Grundmagnet 22 weist zur Erzeugung des homogenen Grundmagnetfelds 30 ein Helmholtz-Spulenpaar 31 mit zwei Magnetspulen 32 auf. Dabei ist eine erste Magnetspule 32 des Helmholtz-Spulenpaares 31 in einem Frontbereich 33 der Magneteinheit 21, insbesondere des Grundmagneten 22, und eine zweite Magnetspulen 32 des Helmholtz-Spulenpaares 31 in einem Heckbereich 34 der Magneteinheit 21, insbesondere des Grundmagneten 22, angeordnet. Der Frontbereich 33 erstreckt sich hierbei 10 cm bis 30 cm von einer Einführöffnung des Patientenaufnahmebereich 25 in den Patientenaufnahmebereich 25 und die Magneteinheit 21 hinein. Der Heckbereich 34 erstreckt sich hierbei 10 cm bis 30 cm von einer Endöffnung des Patientenaufnahmebereich 25 in den Patientenaufnahmebereich 25 und die Magneteinheit 21 hinein. Zwischen den beiden Magnetspulen 32 des Helmholtz-Spulenpaares 31 ist bevorzugt das FoV der Scannereinheit mit dem homogenen Grundmagnetfeld 30 angeordnet.

Die Gradientenspuleneinheit 23 der Magneteinheit 21 ist zu einer Erzeugung von Magnetfeldgradienten, die für eine Ortskodierung während einer Bildgebung verwendet werden, ausgebildet. Die Gradientenspuleneinheit 23 wird mittels einer Gradientensteuereinheit 35 der Magnetresonanzvorrichtung 20 gesteuert. Die Hochfrequenzantenneneinheit 24 der Magneteinheit 21 ist zu einer Anregung einer Polarisation, die sich in dem von dem Grundmagneten 22 erzeugten Grundmagnetfeld 30 einstellt, ausgebildet. Die Hochfrequenzantenneneinheit 24 wird von einer Hochfrequenzantennensteuereinheit 36 der Magnetresonanzvorrichtung 20 gesteuert und strahlt hochfrequente Magnetresonanzsequenzen in den Patientenaufnahmebereich 25 der Magnetresonanzvorrichtung 20 ein.

Zu einer Steuerung des Grundmagneten 22, der Gradientensteuereinheit 35 und zur Steuerung der Hochfrequenzantennensteuereinheit 36 weist die Magnetresonanzvorrichtung 20 eine Magnetresonanzsteuereinheit 37 auf. Die Magnetresonanzsteuereinheit 37 steuert zentral die Magnetresonanzvorrichtung 20, wie beispielsweise das Durchführen einer vorbestimmten bildgebenden Gradientenechosequenz. Zudem umfasst die Magnetresonanzsteuereinheit 37 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 20 eine Benutzerschnittstelle 38, die mit der Magnetresonanzsteuereinheit 37 verbunden ist. Steuerinformationen wie beispielsweise Bildgebungsparameter, sowie rekonstruierte Magnetresonanzbilder können auf einer Darstellungseinheit 39, beispielsweise auf zumindest einem Monitor, der Benutzerschnittstelle 38 für ein medizinisches Bedienpersonal angezeigt werden. Weiterhin weist die Benutzerschnittstelle 38 eine Eingabeeinheit 40 auf, mittels der Informationen und/oder Parameter während eines Messvorgangs von einem medizinischen Bedienpersonal eingegeben werden können.

Die dargestellte Magnetresonanzvorrichtung 20 kann selbstverständlich weitere Komponenten umfassen, die Magnetresonanzvorrichtungen 20 gewöhnlich aufweisen. Eine allgemeine Funktionsweise einer Magnetresonanzvorrichtung 20 ist zudem dem Fachmann bekannt, so dass auf eine detaillierte Beschreibung der weiteren Komponenten verzichtet wird.

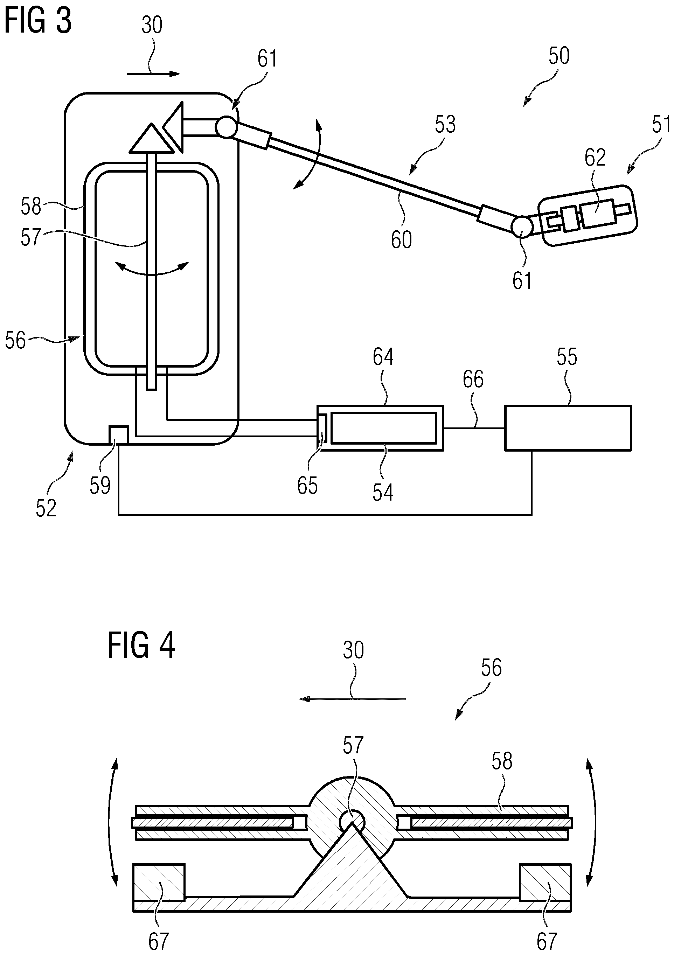

Die Elastographievorrichtung 50 des Magnetresonanzsystems 10 ist zu einer Anregung eines zu untersuchenden Bereichs des Patienten 26 während einer Magnetresonanz-Elastographie-Untersuchung ausgebildet. Hierzu umfasst die Elastographievorrichtung 50 eine Vibrationseinheit 51, eine magnetresonanzkompatible Antriebseinheit 52 und eine Übertragungseinheit 53. Zudem weist die Elastographievorrichtung 50 eine Motortreibereinheit 54 und eine Steuereinheit 55 auf (Fig. 1 und 3) .

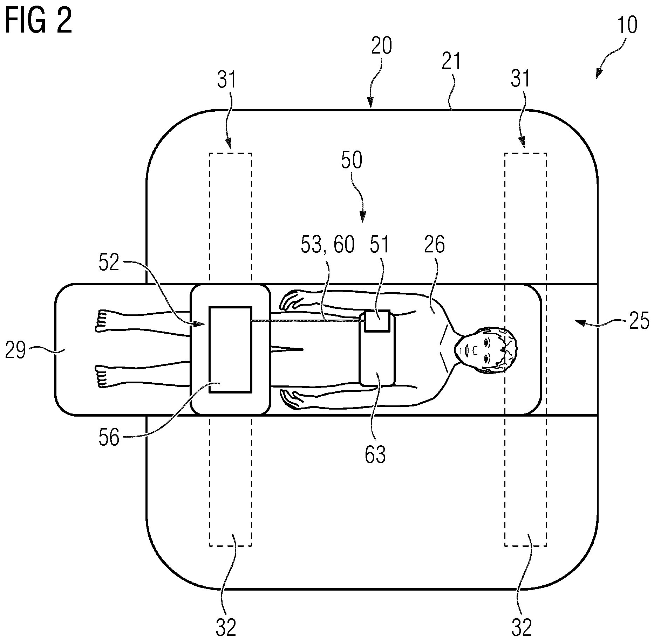

Die magnetresonanzkompatible Antriebseinheit 52 ist zu einer Erzeugung und/oder Generierung eines Antriebsmoments für die Vibrationseinheit 51 ausgebildet und weist hierzu einen magnetresonanzkompatiblen Motor 56 auf. Der magnetresonanzkompatibel Motor 56 ist dabei innerhalb des von dem Grundmagneten 22 erzeugten Grundmagnetfelds 30 angeordnet. Zudem umfasst der magnetresonanzkompatible Motor 56 einen Stator, wobei der Stator eine dominante Komponente des Grundmagnetfelds 30 des Grundmagneten 22 umfasst. Innerhalb des Patientenaufnahmebereichs 25 und/oder nahe am Isozentrum umfasst das Grundmagnetfeld 30 des Grundmagneten 22 nur eine dominante Komponente B₀ in z-Richtung der Magnetresonanzvorrichtung 20 (Fig. 1 und 2). Auch außerhalb des FoVs und/oder außerhalb des Patientenaufnahmebereichs 25 ist die dominante Komponente des Grundmagnetfelds 30 und/oder eines Streufelds bevorzugt in z-Richtung der Magnetresonanzvorrichtung 20 ausgerichtet. Diese dominante Komponente des Grundmagnetfelds 30 dient der magnetresonanzkompatiblen Antriebseinheit 52, insbesondere dem elektromagnetischen und magnetresonanzkompatiblen Motor 56, als Stator. Dabei ist die dominanten Komponenten des Grundmagnetfelds 30 senkrecht zu einer Motorachse 57 des magnetresonanzkompatiblen Motors 56 ausgerichtet (Fig. 3 und 4).

Der magnetresonanzkompatible Motor 56 umfasst zudem einen Rotor und/oder ein drehbares Motorelement 58 (Fig. 3). Der Rotor und/oder das drehbare Motorelement 58 umfasst zumindest ein drehbar gelagertes Spulenelement mit einer senkrecht zur dominanten Komponente des Grundmagnetfelds 30 ausgerichteten, von der Motorachse 57 gebildeten Spulenachse. Dabei weist das zumindest eine drehbar gelagerte Motorelement 58, insbesondere das drehbar gelagerte Spulenelement, des magnetresonanzkompatiblen Motors 56 eine Kupferdrahtspule mit mehreren Spulenwicklungen auf. Das drehbar gelagerte Motorelement 58 ist zur Generierung eines Antriebsmoments des magnetresonanzkompatiblen Motors 56 ausgebildet. Eine Drehbewegung des drehbar gelagerten Motorelements 58 kann dabei auch nur eine Teildrehung und keine komplette Drehung um die Motorachse 57 umfassen. Vorzugsweise ist das zumindest eine drehbar gelagerte Motorelement 58 in beide Richtungen um die Motorachse 57 drehbar gelagert (Fig. 4). Dabei wirkt auf das drehbar gelagert Motorelement 58 eine Lorentzkraft, die eine Drehung und damit eine Generierung des Antriebsmoments bewirkt. Bei einer Drehung des zumindest einen drehbar gelagerten Motorelements 58 um die Motorachse 57, insbesondere der Spulenachse, ändert sich dabei eine Neigung der Spulenfläche bezüglich des Grundmagnetfelds 30 und/oder der dominanten Komponente des Grundmagnetfelds 30. Eine Drehrichtung und/oder Rotationsrichtung des drehbar gelagerten Motorelements 58 ist dabei abhängig von einer Stromrichtung eines durch das drehbar gelagerten Motorelement 58 fließenden Stroms (Fig. 3 und 4). Zur Begrenzung einer Drehung in eine Richtung weist der magnetresonanzkompatible Motor 56 zwei Stoppelemente 67 auf, wobei ein erstes Stoppelement 67 die Drehbewegung des drehbar gelagerten Motorelements 58 in eine erste Drehrichtung begrenzt und ein zweites Stoppelement 67 die Drehbewegung des drehbar gelagerten Motorelements 58 in eine erste Drehrichtung begrenzt.

Für eine Ausnutzung einer maximalen Feldstärke des Grundmagnetfelds 30, insbesondere der dominanten Komponente des Grundmagnetfelds 30, ist die magnetresonanzkompatible Antriebseinheit 52, insbesondere der magnetresonanzkompatible Motor 56, innerhalb des Patientenaufnahmebereichs 25 in einem von einer Magnetspule 32 des Helmholtz-Spulenpaares 31 des Grundmagneten 30 überdeckten Bereichs angeordnet. vorteilhafterweise ist die magnetresonanzkompatible Antriebseinheit 52, insbesondere der magnetresonanzkompatible Motor 56, an einer gleichen Position in z-Richtung wie eine Magnetspule 32 des Helmholtz-Spulenpaares 31 des Grundmagneten 30 angeordnet, wie dies in Fig. 2 einer schematischen Anordnung der Vibrationseinheit 51 und des magnetresonanzkompatiblen Motors 56 am Patienten 26 innerhalb des Patientenaufnahmebereichs 25 zu sehen ist. Weist beispielsweise das von dem Helmholtz-Spulenpaar 31 erzeugte homogene Grundmagnetfeld 30 im FoV, insbesondere zwischen den beiden Magnetspulen 32 des Helmholtz-Spulenpaares 31 eine Magnetfeldstärke von 3,0 T auf, so umfasst die maximale Magnetfeldstärke an der Position einer der beiden Magnetspulen 32 des Helmholtz-Spulenpaares 31 3,6 T.

Im vorliegenden Ausführungsbeispiel weist der magnetresonanzkompatible Motor 56 zusätzlich einen Magnetfeldsensor 59, beispielweise einen Hallsensor, auf, um eine Magnetfeldstärke an der Position des magnetresonanzkompatiblen Motors 56 zu erfassen. Ein derartiger Magnetfeldsensor 59 ist jedoch optional und nicht zwingend erforderlich, sofern der magnetresonanzkompatible Motor 56 an einer Position mit bekannter Magnetfeldstärke des Grundmagnetfelds 30, insbesondere der dominanten Komponente des Grundmagnetfelds 30, während einer Magnetresonanz-Elastographie-Untersuchung positioniert ist.

Die Übertragungseinheit 53 ist zu einer Übertragung des von der magnetresonanzkompatiblen Antriebseinheit 52, insbesondere des magnetresonanzkompatiblen Motors 56, generierten Antriebsmoment auf die Vibrationseinheit 51 ausgebildet. Hierzu weist die Übertragungseinheit 53 eine Antriebswelle 60 auf. Die Antriebswelle 60 ist dabei in ihrer Länge variabel ausgebildet. Die in ihrer Länge variable Antriebswelle 60 kann dabei teleskopartig ausgebildet sein. Beispielsweise kann die in ihrer Länge variable Antriebswelle 60 zwei oder mehr ineinandergreifende Stangen, beispielsweise Stangen mit einem viereckigem oder einem sechseckigem Querschnitt, umfassen. Vorzugsweise weisen hierbei die ineinandergreifenden Stangen unterschiedliche Querschnitte auf. In Fig. 3 weist die Antriebswelle 60 zudem zwei Gelenke 61 auf, beispielsweise Kardangelenke, wobei in Fig. 3 die Antriebswelle 60 beispielhaft mit zwei Gelenken 61 dargestellt ist. Eine Ausbildung der Antriebswelle 60 mit mehr als zwei Gelenken 61 ist dabei jederzeit möglich. Alternativ zu einer Ausbildung der Antriebswelle 60 mit Gelenken 61 kann die Antriebswelle 60 auch biegsam ausgebildet sein.

Die Vibrationseinheit 51 weist ein Vibrationselement 62 auf das zur Erzeugung von Vibrationen und/oder Schwingungen und zu einer Übertragung dieser Vibrationen und/oder Schwingungen auf den Patienten 26 ausgebildet ist. Hierzu ist die Vibrationseinheit 51, insbesondere das Vibrationselement 62, am Patienten 26 an dem zu untersuchenden Bereich des Patienten 26 positioniert. Dabei kann die Vibrationseinheit 52 zudem einen Befestigungsgurt 63 aufweisen, wobei mittels des Befestigungsgurts 63 die Vibrationseinheit 52, insbesondere das Vibrationselement 62, an dem zu untersuchenden Bereich des Patienten 26 befestigt wird. Das Vibrationselement 62 umfasst bevorzugt eine Schwingmasse und/oder Vibrationsmasse, die zu einer Erzeugung einer Schwingung und/oder Vibration ausgebildet ist. Zudem ist das Vibrationselement 62 dazu ausgebildet, die erzeugte Schwingung und/oder Vibration auf den Patienten 26, insbesondere auf den zu untersuchenden Bereich zu übertragen.

Das von der magnetresonanzkompatiblen Antriebseinheit 52, insbesondere von dem magnetresonanzkompatiblen Motor 56, erzeugte Antriebsmoment kann dabei mittels der Übertragungseinheit 53, insbesondere der Antriebswelle 60, direkt auf das Vibrationselement 62 übertragen werden (Fig. 2 und 3). Zudem kann es auch sein, dass das Vibrationselement 62 als Exzenterelement ausgebildet ist, wobei auf das Exzenterelement das von dem magnetresonanzkompatiblen Motor 56 erzeugte Antriebsmoment mittels der Übertragungseinheit 53, insbesondere der Antriebswelle 60, übertragbar ist. Dabei kann die Vibrationseinheit 51 zudem eine Sperrklinke, die zu dem Exzenterelement vorgeschaltet angeordnet ist, umfassen, so dass stets ein Drehmoment in eine gleiche Richtung auf das Exzenterelement für eine Generierung eine Vibration und/oder Schwingung übertragen wird.

Die Motortreibereinheit 54 der Elastographievorrichtung 50 ist bevorzugt in einem Schirmgehäuse 64 der Elastographievorrichtung 50 angeordnet (Fig. 3). Das Schirmgehäuse 64 schirmt die Motortreibereinheit 54 hinsichtlich einer Hochfrequenzstrahlung von der Magneteinheit ab. Vorteilhaft umfasst das Schirmgehäuse 64 ein elektromagnetisches Filterelement 65, wobei das elektromagnetisches Filterelement 65 alle ausgehenden Signale der Motortreibereinheit 54 bezüglich HF filtert. Die Motortreibereinheit 54 umfasst insbesondere eine Schaltung und/oder eine Schaltungseinheit zur Ansteuerung des magnetresonanzkompatiblen Motors 56. Bevorzugt umfasst die Motortreibereinheit 54 eine H-Brücke zur Spannungsregelung des magnetresonanzkompatiblen Motors 56. Aufgrund der Anordnung der Motortreibereinheit 54 innerhalb des Schirmgehäuses 64 kann die Motortreibereinheit 54 sowohl innerhalb des Patientenaufnahmebereichs 25 als auch außerhalb des Patientenaufnahmebereichs 25 angeordnet sein. Im vorliegenden Ausführungsbeispiel ist die Motortreibereinheit 54 innerhalb des Patientenaufnahmebereichs 25 und/oder in einem Streufeldbereich des Grundmagnetfelds 30 angeordnet (Fig. 2).

Zur Ansteuerung des magnetresonanzkompatiblen Motors 56 wird von der Motortreibereinheit 54, insbesondere der H-Brücke, ein definierte Strom für den magnetresonanzkompatiblen Motor 56 bereitgestellt und direkt an den magnetresonanzkompatiblen Motor 56 übertragen. Derartige Ströme für einen Betrieb des magnetresonanzkompatiblen Motors 56 und damit zur Generierung eines Antriebsmoments für die Vibrationseinheit können dabei eine Stromstärke zwischen 0,5 A und maximal 10 A aufweisen.

Die Steuereinheit 55 der Elastographievorrichtung 50 ist zu einer Steuerung der Elastographievorrichtung 50 ausgebildet. Die Steuereinheit 55 sendet in einem Betriebsmodus der Elastographievorrichtung 50 Steuersignale direkt an die Motortreibereinheit 54 und steuert somit die Elastographievorrichtung 50. Zu einer Übertragung von Steuersignalen zwischen der Steuereinheit 54 und der Motortreibereinheit 54 weist die Elastographievorrichtung 50 eine optische Verbindungseinheit 66 auf (Fig. 3). Die optische Verbindungseinheit 66 umfasst bevorzugt optische Leiter, beispielsweise Lichtleiter und/oder Glasfaserkabel. Alternativ zu einer optischen Übertragungseinheit 66 kann die Übertragungseinheit 66 auch galvanisch oder über Funk erfolgen.

Die Steuereinheit 55 ist zudem dazu ausgebildet, die Elastographievorrichtung 50 mit einer Messsequenz der Magnetresonanzvorrichtung 20 während einer Magnetresonanz-Elastographie-Untersuchung zu synchronisieren. Bevorzugt steuert die Steuereinheit 55 die Motortreibereinheit 54 derart, dass ein Schwingungsprozess und/oder Anregungsprozess synchron mit der auszuspielenden Messsequenz der Magnetresonanzvorrichtung 20 während einer Magnetresonanz-Elastographie-Untersuchung ist.

Bevorzugt wird hierbei ein Anfang und eine Ende der Messsequenz auf den Schwingungsprozess und/oder Anregungsprozess abgestimmt. Zur Synchronisierung mit der Messsequenz kann die Steuereinheit 55 der Elastographievorrichtung 50 auch zumindest ein Triggersignal der Magnetresonanzsequenzsteuereinheit 37 bereitstellen, so dass die Messsequenz getriggert durch die Elastographievorrichtung 50 gestartet wird. Dabei kann für die Elastographievorrichtung 50 auch ein Einschwingvorgang vorgesehen sein, bis die Mechanik, insbesondere der magnetresonanzkompatible Motor 56 und die Vibrationseinheit 51, sich auf eine Anregungsfrequenz eingeschwungen haben. Dieser Einschwingvorgang wird bevorzugt von der Steuereinheit 55 bei der Bereitstellung des Triggersignals berücksichtigt. Ein Anregungsvorgang und/oder ein Schwingungsvorgang für eine Anregung mittels der Elastographievorrichtung 50 umfasst bevorzugt eine Frequenz zwischen 50 Hz und 1000 Hz. Bevorzugt umfasst eine Anregungsfrequenz und/oder eine Schwingungsfrequenz ca. 100 Hz.

Weist die Elastographievorrichtung 50, insbesondere der magnetresonanzkompatible Motor 56, einen Magnetfeldsensor 59 auf, so werden die von dem Magnetfeldsensor 59 erfassten Daten an die Steuereinheit 55 übertragen. Anhand der erfassten Magnetfeldstärke kann von der Steuereinheit 55 ein durch den magnetresonanzkompatiblen Motor 56 zu fließender Strom ermittelt und von der Motortreibereinheit 54 eingestellt werden, um ein vorteilhaftes Antriebsmoment für die Vibrationseinheit 51 zu erhalten.

Die dargestellte Elastographievorrichtung 50 kann selbstverständlich weitere Komponenten umfassen, die Elastographievorrichtungen 50 gewöhnlich aufweisen. Eine allgemeine Funktionsweise einer Elastographievorrichtung 50 ist zudem dem Fachmann bekannt, so dass auf eine detaillierte Beschreibung der weiteren Komponenten verzichtet wird.

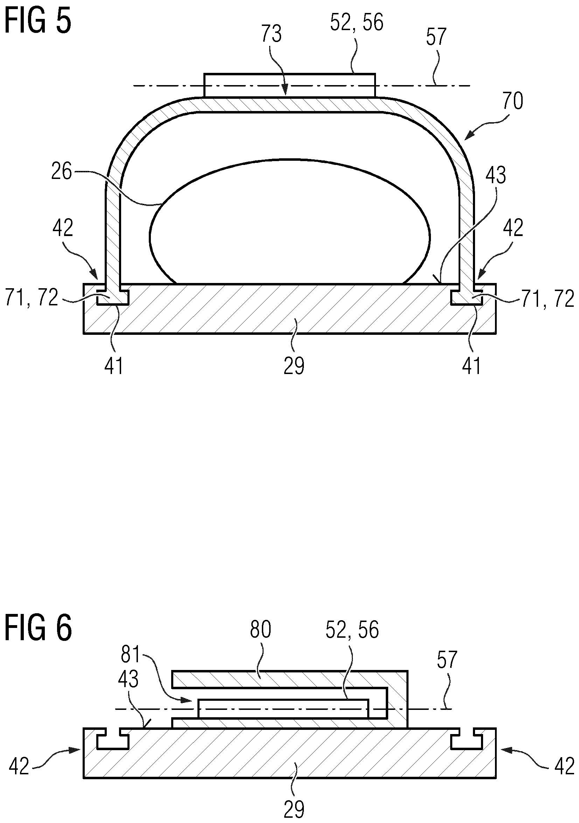

In Fig. 5 ist ein erstes Ausführungsbeispiel für eine Anordnung und/oder Positionierung der Elastographievorrichtung 50 für eine Magnetresonanz-Elastographie-Untersuchung dargestellt. Im Wesentlichen gleichbleibende Bauteile, Merkmale und Funktionen des Magnetresonanzsystems 10, insbesondere der Magnetresonanzvorrichtung 20 und der Elastographievorrichtung 50, sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 bis 4, wobei bezüglich gleichbleibender Bauteile, Merkmale und Funktionen auf die Beschreibung des Ausführungsbeispiels in den Fig. 1 bis 4 verwiesen wird.

Eine Ausgestaltung der magnetresonanzkompatiblen Antriebseinheit 52, der Vibrationseinheit 51, der Übertragungseinheit 53, der Motortreibereinheit 54 und der Steuereinheit 55 der Elastographievorrichtung 50 entsprechen den Ausführungen zu den Fig. 1 bis 4, auf die hiermit verwiesen wird.

Für eine sichere und stabile Anordnung und/oder Positionierung der magnetresonanzkompatiblen Antriebseinheit 52, insbesondere des magnetresonanzkompatiblen Motors 56, weist die Elastographievorrichtung 50 im vorliegenden Ausführungsbeispiel eine Haltevorrichtung 70 auf. Die Haltevorrichtung 70 ist zu einer lösbaren Anordnung und/oder Positionierung an dem Patiententisch 29 der Patientenlagerungsvorrichtung 27 ausgebildet.

Der Patiententisch 29 weist zwei Befestigungsschienen 41 auf, die sich jeweils in Längsrichtung des Patiententischs 29 erstrecken. Die beiden Befestigungsschienen 41 sind dabei jeweils an einem seitlichen Randbereich 43 des Patiententischs 29 angeordnet, wobei zwischen den beiden seitlichen Randbereichen 42 und damit den beiden Befestigungsschienen 41 des Patiententischs 29 eine Lagerungsfläche 43 und/oder ein Lagerungsbereich des Patiententischs 29 zur Lagerung des Patienten 26 angeordnet ist. Mittels der beiden Befestigungsschienen 41 können Zubehöreinheiten, die für eine anstehende Magnetresonanzuntersuchung und/oder eine anstehende Magnetresonanz-Elastographie-Untersuchung erforderlich sind, sicher am Patiententisch 29 befestigt und/oder angeordnet werden.

In Fig. 5 ist ein Schnitt durch den Patiententisch 29 mit der an dem Patiententisch 29 angeordneten Haltevorrichtung 70 dargestellt. Zur Befestigung der Haltevorrichtung 70 an den beiden Befestigungsschienen 41 des Patiententischs 29 ist die Haltevorrichtung 70 als konvexer Haltebogen ausgebildet. Dieser konvexe Haltebogen umfasst dabei zwei Endbereiche 71, wobei die beiden Endbereiche 71 in Längserstreckung des konvexen Haltebogens an gegenüberliegenden Seiten angeordnet sind. Die beiden Endbereiche 71 umfassen jeweils ein Befestigungselement 72 zur Befestigung des konvexen Haltebogens an der jeweiligen Befestigungsschiene 41. Zudem umfasst der konvexe Haltebogen einen mittleren Befestigungsbereich 73, wobei der mittlere Befestigungsbereich 73 zu einer Anordnung und/oder Befestigung des magnetresonanzkompatiblen Motors 56 ausgebildet ist. Befindet sich der konvexe Haltebogen an dem Patiententisch 29 angeordnet, wölbt sich der konvexe Haltebogen über die Lagerungsfläche 43 und den Lagerungsbereich des Patiententischs 29. Somit wölbt sich der konvexe Haltebogen auch über dem auf dem Patiententisch 29 positionierten Patienten 26.

Bevorzugt ist der konvexe Haltebogen derart an dem Patiententisch 29 positioniert, dass in einer Untersuchungsposition des Patiententischs 29 der konvexe Haltebogen und damit der an dem konvexen Haltebogen befestigte magnetresonanzkompatible Motor 56 an einer z-Position innerhalb des Patientenaufnahmebereichs 26 positioniert ist, an der auch eine Magnetspule 32 des Helmholtz-Spulenpaares 31 des Grundmagneten 22 angeordnet ist. Zudem ist insbesondere der Befestigungsbereich 73 derart ausgebildet, dass eine Motorachse 57, insbesondere eine Drehachse, des magnetresonanzkompatiblen Motors 56 senkrecht zur dominanten Komponenten des Grundmagnetfelds 30 ausgerichtet ist, wenn der magnetresonanzkompatible Motor 56 an dem konvexen Haltebogen positioniert ist und der konvexe Haltebogen am Patiententisch 29 positioniert ist.

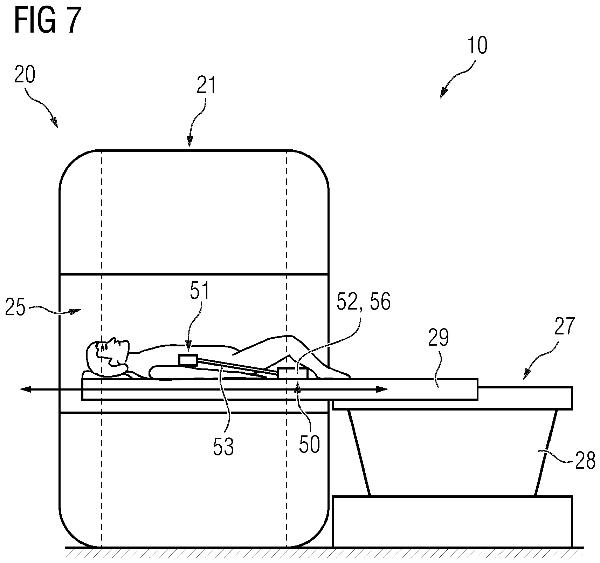

In Fig. 6 und 7 ist ein zweites Ausführungsbeispiel für eine Anordnung und/oder Positionierung der Elastographievorrichtung 50 für eine Magnetresonanz-Elastographie-Untersuchung dargestellt. Im Wesentlichen gleichbleibende Bauteile, Merkmale und Funktionen des Magnetresonanzsystems 10, insbesondere der Magnetresonanzvorrichtung 20 und der Elastographievorrichtung 50, sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 bis 4, wobei bezüglich gleichbleibender Bauteile, Merkmale und Funktionen auf die Beschreibung des Ausführungsbeispiels in den Fig. 1 bis 4 verwiesen wird.

Eine Ausgestaltung der magnetresonanzkompatiblen Antriebseinheit 52, der Vibrationseinheit 51, der Übertragungseinheit 53, der Motortreibereinheit 54 und der Steuereinheit 55 der Elastographievorrichtung 50 entsprechen den Ausführungen zu den Fig. 1 bis 4, auf die hiermit verwiesen wird.

Die Magnetresonanzvorrichtung 20 in Fig. 6 und 7 umfasst ein Positionierungskissen 80, das zur Lagerung und/oder Positionierung des Patienten 26 während einer Magnetresonanz-Elastographie-Untersuchung ausgebildet ist. Beispielsweise kann ein derartiges Positionierungskissen 80 zur einer Lagerung und/oder Positionierung von Knieen, insbesondere zur Unterlage für Kniee, des Patienten 26 verwendet werden. Das Positionierungskissen 80 weist dabei eine Tasche und/oder einen Aufnahmebereich 81 auf, die und/oder der zur Aufnahme des magnetresonanzkompatiblen Motors 56 der Elastographievorrichtung 50 ausgebildet ist. Bevorzugt ist die Tasche und/oder der Aufnahmebereich 81 derart ausgebildet, dass eine Motorachse 57, insbesondere eine Drehachse, des magnetresonanzkompatiblen Motors 56 senkrecht zur dominanten Komponenten des Grundmagnetfelds 30 ausgerichtet ist, wenn der magnetresonanzkompatible Motor 56 innerhalb der Tasche und/oder des Aufnahmebereichs 81 des Positionierungskissens 80 positioniert ist und das Positionierungskissen auf dem Patiententisch 29 positioniert ist (Fig. 7). In Fig. 6 ist ein Schnitt durch den Patiententisch 29 mit dem an dem Patiententisch 29 angeordneten Positionierungskissen 80 dargestellt. Fig. 7 zeigt eine Seitenansicht des Patiententischs 29 mit einem auf dem Patiententisch 29 positionierten Patienten 26 und einer Elastographievorrichtung 50. Neben des innerhalb des Positionierungskissens 80 positionierten und/oder angeordneten magnetresonanzkompatiblen Motors 56 ist in Fig. 7 auch die Übertragungseinheit 53 und die Vibrationseinheit 51, die an dem zu untersuchenden Bereich des Patienten 26 positioniert ist, dargestellt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzsystem umfassend:
- eine Magnetresonanzvorrichtung mit einer Scannereinheit, die einen Grundmagneten, eine Gradientenspuleneinheit und eine Hochfrequenzantenneneinheit aufweist, und einem von der Scannereinheit zumindest teilweise umgebenen Patientenaufnahmebereich, und
- eine Elastographievorrichtung, die zu einer Anregung von zu untersuchenden Bereichen eines Patienten während einer Magnetresonanz-Elastographie-Untersuchung am Patienten ausgebildet ist, umfassend eine Vibrationseinheit, eine magnetresonanzkompatiblen Antriebseinheit und eine Übertragungseinheit zu einem Übertragen eines von der magnetresonanzkompatiblen Antriebseinheit erzeugten Antriebsmoments auf die Vibrationseinheit,
**dadurch gekennzeichnet, dass** die magnetresonanzkompatibel Antriebseinheit einen magnetresonanzkompatiblen Motor aufweist.

2. Magnetresonanzsystem nach Anspruch 1,
**dadurch gekennzeichnet, dass** der magnetresonanzkompatible Motor innerhalb eines von dem Grundmagneten erzeugten homogenen Grundmagnetfelds angeordnet ist, wobei der magnetresonanzkompatible Motor einen Stator umfasst und der Stator eine dominante Komponente des Grundmagnetfelds des Grundmagneten umfasst.

3. Magnetresonanzsystem nach einem der vorhergehenden Ansprüche,
**dadurch gekennzeichnet, dass** der Grundmagnet eine Magnetspule zur Erzeugung des homogenen Grundmagnetfelds aufweist, wobei der magnetresonanzkompatible Motor zur Erzeugung des Antriebsmoments für die Vibrationseinheit innerhalb des Patientenaufnahmebereichs in einem von der Magnetspule überdeckten Bereich angeordnet ist.

4. Magnetresonanzsystem nach einem der vorhergehenden Ansprüche,
**dadurch gekennzeichnet, dass** der magnetresonanzkompatible Motor einen Magnetfeldsensor aufweist.

5. Magnetresonanzsystem nach einem der vorhergehenden Ansprüche,
**dadurch gekennzeichnet, dass** die Elastographievorrichtung eine Haltevorrichtung aufweist, an der der magnetresonanzkompatible Motor während einer Magnetresonanz-Elastographie-Untersuchung angeordnet ist.

6. Magnetresonanzsystem nach Anspruch 5,
**dadurch gekennzeichnet, dass** die Magnetresonanzvorrichtung eine Patientenlagerungsvorrichtung mit einem innerhalb des Patientenaufnahmebereich bewegbaren Patiententisch aufweist, wobei die Haltevorrichtung zu einer lösbaren Befestigung an dem Patiententisch ausgebildet ist.

7. Magnetresonanzsystem nach einem der Ansprüche 5 oder 6, **dadurch gekennzeichnet, dass** die Haltevorrichtung einen konvexen Haltebogen mit zwei Endbereichen und einem mittleren Befestigungsbereich umfasst, wobei die beiden Endbereiche zu einer lösbaren Befestigung an dem Patiententisch ausgebildet sind und der mittlere Befestigungsbereich für eine Anordnung des magnetresonanzkompatiblen Motors ausgebildet ist.

8. Magnetresonanzsystem nach einem der vorhergehenden Ansprüche,
**dadurch gekennzeichnet, dass** die Magnetresonanzvorrichtung ein Positionierungskissen zu einer Positionierung eines Patienten aufweist, wobei der magnetresonanzkompatible Motor innerhalb des Positionierungskissens angeordnet ist.

9. Magnetresonanzsystem nach einem der vorhergehenden Ansprüche,
**dadurch gekennzeichnet, dass** die Übertragungseinheit eine in ihrer Länge variable Antriebswelle aufweist.

10. Magnetresonanzsystem nach einem der vorhergehenden Ansprüche,
**dadurch gekennzeichnet, dass** die Vibrationseinheit ein Vibrationselement aufweist, wobei das von dem magnetresonanzkompatiblen Motor erzeugte Antriebsmoment auf das Vibrationselement übertragbar ist.

11. Magnetresonanzsystem nach einem der vorhergehenden Ansprüche,
**dadurch gekennzeichnet, dass** die Vibrationseinheit ein Exzenterelement aufweist, wobei auf das Exzenterelement das von dem magnetresonanzkompatiblen Motor erzeugte Antriebsmoment übertragbar ist.

12. Magnetresonanzsystem nach einem der vorhergehenden Ansprüche,
**dadurch gekennzeichnet, dass** die Elastographievorrichtung eine Motortreibereinheit und ein Schirmgehäuse umfasst, wobei die Motortreibereinheit in dem Schirmgehäuse angeordnet ist.

13. Magnetresonanzsystem nach einem der vorhergehenden Ansprüche,
**dadurch gekennzeichnet, dass** die Elastographievorrichtung eine Steuereinheit aufweist, die zu einer Synchronisierung der Elastographievorrichtung mit einer Messsequenz der Magnetresonanz- Elastographie-Untersuchung ausgebildet ist.

14. Magnetresonanzsystem nach Anspruch 12 und 13,
**dadurch gekennzeichnet, dass** die Elastographievorrichtung eine optische Übertragungseinheit umfasst, die zwischen der Motortreibereinheit und der Steuereinheit angeordnet ist.
